# EUROPEAN PATENT SPECIFICATION

(11) **EP 4 285 759 B1**
(45) Date of publication and mention of the grant of the patent: **17.06.2026**
(21) Application number: 23175248.6
(22) Date of filing: 25.05.2023
(51) Int. Cl.: A24F 40/465, A24F 40/10

(54) **VAPORIZATION ASSEMBLY AND ELECTRONIC VAPORIZER**
VERDAMPFUNGSANORDNUNG UND ELEKTRONISCHER VERDAMPFER
ENSEMBLE DE VAPORISATION ET VAPORISATEUR ÉLECTRONIQUE

(30) Priority: 02.06.2022 CN 202210620385
(43) Date of publication of application: 06.12.2023
(73) Proprietor: Shenzhen Smoore Technology Limited, Shenzhen, Guangdong 518102 (CN)
(72) Inventor: LUO, Yongjie, Shenzhen, Guangdong, 518102 (CN); YANG, Baomin, Shenzhen, Guangdong, 518102 (CN); FAN, Jichang, Shenzhen, Guangdong, 518102 (CN); WEN, Zhihua, Shenzhen, Guangdong, 518102 (CN)
(74) Representative: Manitz Finsterwald Patent- und Rechtsanwaltspartnerschaft mbB

(56) References cited:
- WO-A1-2020/194297
- WO-A1-2023/125715

## Description

### TECHNICAL FIELD

The present application relates to the field of vaporization technologies, and in particular, to a heating unit, a vaporization assembly, and an electronic vaporizer.

### BACKGROUND

An aerosol is a colloidal dispersion system formed by dispersing small solid or liquid particles and suspending the particles in a gas medium. Since the aerosol can be absorbed by a human body through a respiratory system, users are provided with a new alternative absorption manner. A vaporizer refers to a device that forms an aerosol from a stored vaporizable medium by means of heating or ultrasound. The vaporizable medium includes e-liquids containing nicotine, medical drugs, skin care lotions, etc. Vaporization of the mediums can deliver inhalable aerosols to the users, replacing conventional product forms and absorption manners. Related technology is known from WO 2020/194297 A1.

A conventional electronic vaporizer mainly heats an aerosol-generating substrate by resistance heating. When resistance heating is replaced with electromagnetic vaporization, an original configuration of the vaporizer cannot meet requirements of electromagnetic vaporization.

### SUMMARY

Based on the above, there is a need to provide a heating unit, a vaporization assembly, and an electronic vaporizer with respect to the problem that the configuration of the conventional electronic vaporizer is not suitable for electromagnetic vaporization applications. The heating unit, the vaporization assembly, and then electronic vaporizer can achieve a technical effect of meeting the requirements of electromagnetic vaporization. The present invention is set out in the appended claims.

According to the invention, a vaporization assembly comprising a heating unit is provided, including: a heating shell provided with a shell accommodating cavity; a sheet-shaped heating element housed in the shell accommodating cavity and dividing the shell accommodating cavity into a liquid guiding cavity and an airflow channel, the liquid guiding cavity and the airflow channel being located respectively on the two sides of the sheet-shaped heating element in the thickness direction; and a liquid guiding member having one end housed in the liquid guiding cavity and contacting the sheet-shaped heating element, and another end extending out of the liquid guiding cavity.

In an embodiment, the heating shell includes a heating element base and a heating element fixed cover, the heating element fixed cover is located on the side of the liquid guiding member away from the sheet-shaped heating element and fitted to the heating element base, the airflow channel is formed between the heating element base and the sheet-shaped heating element, and the liquid guiding cavity is formed between the heating element fixed cover and the sheet-shaped heating element.

In an embodiment, the side of the heating element fixed cover facing the liquid guiding cavity is provided with a diversion groove.

In an embodiment, the sheet-shaped heating element is provided with a heating element air outlet communicating with the liquid guiding cavity and the airflow channel.

According to the invention, the vaporization assembly includes a liquid storage tank housing provided with an air outlet channel and a liquid storage cavity surrounding the air outlet channel. The heating unit is fitted to an end of the liquid storage tank housing, the air outlet channel communicates with the airflow channel, the liquid guiding cavity communicates with the liquid storage cavity, and the end of the liquid guiding member extending out of the liquid guiding cavity bends and extends into the liquid storage cavity.

In an embodiment, the vaporization assembly further includes an airway sealing member fitted between the liquid storage tank housing and the heating unit. The airway sealing member communicates with the air outlet channel and the airflow channel of the heating unit.

According to the invention, the vaporization assembly further includes a bottom cover for liquid storage tank fitted to an end of the liquid storage tank housing, and the heating unit is housed in the bottom cover.

In an embodiment, the vaporization assembly further includes a sealing member for liquid storage tank located between the bottom cover and the liquid storage tank housing to seal the liquid storage cavity.

In an embodiment, an end of the heating shell away from the liquid storage cavity is provided with a shell air inlet, and an end of the bottom cover away from the liquid storage cavity is provided with a bottom cover air inlet communicating with the shell air inlet.

According to the invention, the vaporization assembly further includes an inductance coil circumferentially surrounding the bottom cover.

According to another aspect of the present application, an electronic vaporizer is provided, including the vaporization assembly described above. The electronic vaporizer further includes a power supply assembly electrically connected to the vaporization assembly and configured to supply power to the vaporization assembly.

In the above heating unit, since an aerosol-generating substrate is heated and vaporized by a thinner sheet-shaped heating element, the sheet-shaped heating element has a faster heating rate than a tubular heating element, thereby achieving an effect of rapid vaporization. Moreover, top feeding is realized through the liquid guiding member, without additionally arranging a liquid inlet channel on a side face of the sheet-shaped heating element to cause the aerosol-generating substrate to enter the liquid guiding member, so a distance between the sheet-shaped heating element and an external coil can be reduced, so as to further increase a vaporization speed.

### BRIEF DESCRIPTION OF THE DRAWINGS

FIG. 1 is a schematic diagram illustrating a vaporization assembly according to an embodiment of the present application.
FIG. 2 is a sectional view of the vaporization assembly shown in FIG. 1 perpendicular to a second direction.
FIG. 3 is a sectional view of the vaporization assembly shown in FIG. 1 perpendicular to a first direction.
FIG. 4 is a schematic exploded view of a partial configuration of the vaporization assembly shown in FIG. 1.
FIG. 5 is a schematic structural diagram illustrating a heating element base according to an embodiment of the present application.
FIG. 6 is a schematic structural diagram illustrating assembly of the heating element base and a sheet-shaped heating element according to an embodiment of the present application.
FIG. 7 is a schematic structural diagram illustrating a heating unit according to an embodiment of the present application.
FIG. 8 is a schematic structural diagram illustrating a heating element fixed cover according to an embodiment of the present application.
FIG. 9 is a schematic diagram illustrating assembly of the heating unit and an airway sealing member according to an embodiment of the present application.

Reference signs:
100: vaporization assembly; 10: liquid storage tank housing; 12: central tube; 121: air outlet channel; 14: housing sidewall; 16: liquid storage cavity; 20: bottom cover; 20a: big end; 20b: small end; 21: bottom cover accommodating cavity; 23: bottom cover air inlet; 30: sealing member; 40: heating unit; 41: heating shell; 412: heating element base; 4121: base bottom wall; 4121a: fixed groove; 4121b: shell air inlet; 4121c: supporting edge; 4122: base top wall; 4122a: shell air outlet; 4123: base front sidewall; 4123a: communicating groove; 4124: base left sidewall; 4125: base right sidewall; 4126: fixed buckle; 414: heating element fixed cover; 4141: limit protrusion; 4143: diversion groove; 43: sheet-shaped heating element; 432: heating element air outlet; 45: liquid guiding member; 47: airflow channel; 50: airway sealing member; 52: sealing member communicating hole; 54: sealing member communicating groove; 60: bottom sealing gasket; 70: inductance coil; 82: coil mounting shell; 84: coil mount; and 90: main shell.

### DETAILED DESCRIPTION OF THE EMBODIMENTS

In order to make the above objectives, features, and advantages of the present application more obvious and understandable, specific implementations of the present application are described in detail below with reference to the accompanying drawings. In the following description, many specific details are set forth in order to fully understand the present application. However, the present application can be implemented in many other ways different from those described herein, and those skilled in the art can make similar improvements without departing from the connotation of the present application. Therefore, the present application is not limited by the specific implementation disclosed below.

In the description of the present application, it should be understood that the orientation or position relationships indicated by the terms "central", "longitudinal", "transverse", "length", "width", "thickness", "up", "down", "front", "back", "left", "right", "vertical", "horizontal", "top", "bottom", "inner", "outer", "clockwise", "counterclockwise", "axial", "radial", "circumferential", etc. are based on the orientation or position relationships shown in the accompanying drawings and are intended to facilitate the description of the present application and simplify the description only, rather than indicating or implying that the device or element referred to must have a particular orientation or be constructed and operated in a particular orientation, and therefore are not to be interpreted as limiting the present application.

In addition, the terms "first" and "second" are used for descriptive purposes only, which cannot be construed as indicating or implying a relative importance or implicitly specifying the number of the indicated technical features. Thus, the features defined with "first" and "second" may explicitly or implicitly include at least one feature. In the description of the present application, "a plurality of" means at least two, such as two or three, unless specifically stated otherwise.

In the description of the present application, unless otherwise specifically stated and limited, the terms "mount," "join," "connect", "fix", etc. should be understood in a broad sense, such as, a fixed connection, a detachable connection, or an integral connection; a mechanical connection, or an electrical connection; a direct connection, an indirect connection through an intermediate medium, internal communication between two elements, or an interaction of two elements, unless otherwise expressly defined. For those of ordinary skill in the art, the specific meanings of the foregoing terms in the present application can be understood on a case-by-case basis.

In the present application, unless otherwise explicitly specified and defined, a first feature being "on" or "under" a second feature may be a case that the first feature is in direct contact with the second feature, or the first feature is in indirect contact with the second feature via an intermediate medium. Furthermore, the first feature being "over", "above" and "on top of" the second feature may be a case that the first feature is directly above or obliquely above the second feature, or only means that the level of the first feature is higher than that of the second feature. The first feature being "below", "underneath" or "under" the second feature may be a case that the first feature is directly underneath or obliquely underneath the second feature, or only means that the level of the first feature is lower than that of the second feature.

It should be noted that when one element is referred to as "fixed to" or "arranged on" another element, it may be directly disposed on the another element or an intermediate element may exist. When one element is considered to be "connected to" another element, it may be directly connected to the another element or an intermediate element may co-exist. The terms "vertical", "horizontal", "up", "down", "left", "right" and similar expressions used herein are for illustrative purposes only, and do not represent unique embodiments.

As described in the background, the aerosol is generated by means of hot boiling vaporization. The local temperature of the vaporized surface may be as high as 350 ° C. The high temperature will lead to local splitting decomposition of the components of the vaporized medium and form carcinogenic harmful substances such as aldehyde, ketone, and nitrile. Especially when the liquid supply is insufficient, the local temperature of the vaporized surface is higher, causing greater harm. Although the existing ultrasonic vaporization technology will not produce harmful substances due to a high temperature, the vaporization effect is limited, the effective vaporization of a high viscosity liquid (≥ 10cp) cannot be completed, and the aerosol particles generated by ultrasonic vaporization are usually ≥ 5µm in the median particle size. For an electronic vaporizer that produces an aerosol for human consumption, the viscosity of the vaporized medium is usually around 300 cp, and in order to ensure a good user experience, the particle size of the aerosol is required to be between 0.5 µm and 3 µm. Therefore, the existing ultrasonic vaporization technology is difficult to meet the requirements of electronic vaporizer.

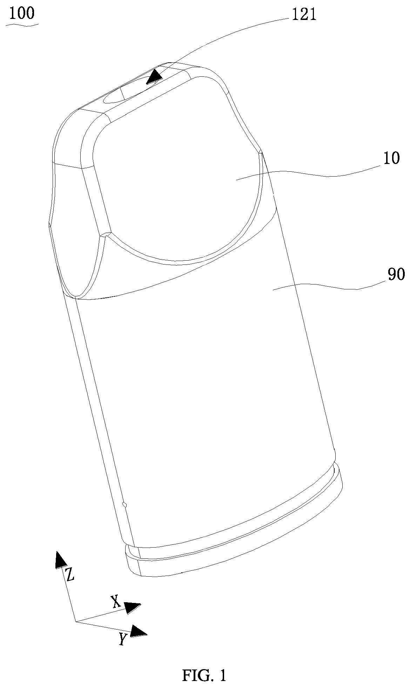

As shown in FIG. 1, an embodiment of the present application provides an electronic vaporizer, including a vaporization assembly 100 and a power supply assembly (not shown) fitted to an end of the vaporization assembly 100. The power supply assembly is fitted to an end of the vaporization assembly 100, electrically connected to the vaporization assembly 100, and configured to supply power to the vaporization assembly 100. In this way, the vaporization assembly 100 heats an aerosol-generating substrate under the action of the power from the power supply assembly to generate an aerosol for a user to inhale. It is to be noted that the configuration of the power supply assembly does not belong to a main innovative point of the present application, and thus is not described in detail therein.

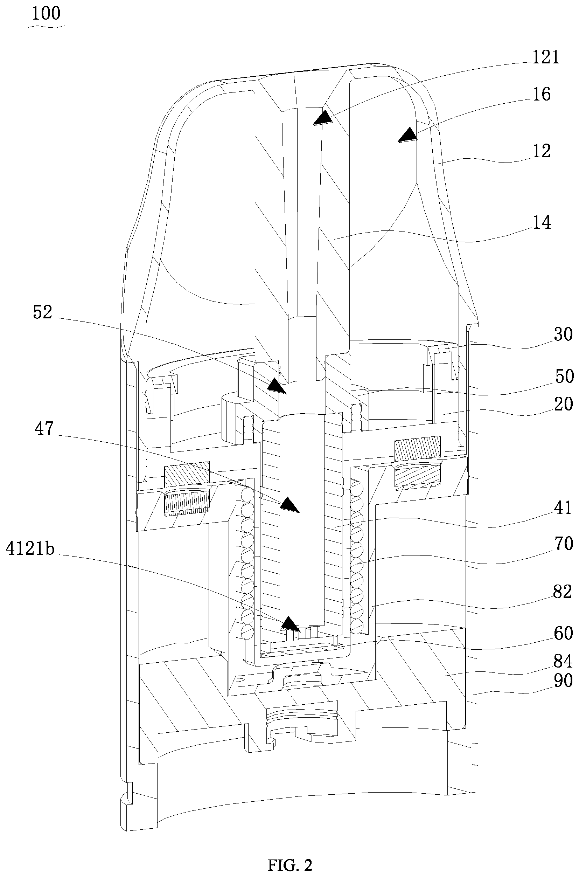

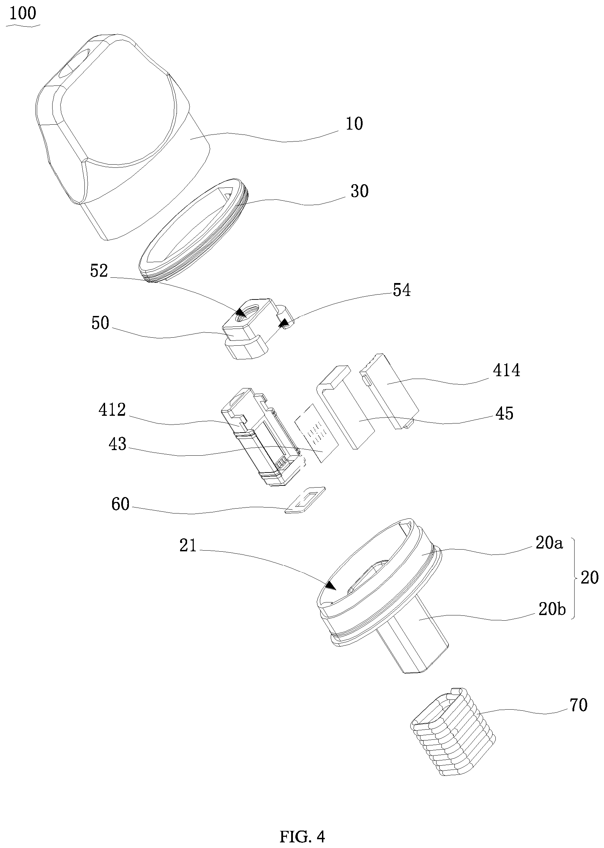

As shown in FIG. 2 to FIG. 4, the vaporization assembly 100 includes a liquid storage tank housing 10, a bottom cover 20 for liquid storage tank, a sealing member 30 for liquid storage tank, and a heating unit 40. The liquid storage tank housing 10 and the bottom cover 20 are sealed and connected through the sealing member 30 to house the heating unit 40.

Specifically, the liquid storage tank housing 10 has a shell-like structure, including a central tube 12 and a housing sidewall 14. The central tube 12 has a roughly hollow cylinder structure, with the interior communicating with an external environment to form an air outlet channel 121. The housing sidewall 14 extends obliquely from the top of the central tube 12 toward the bottom of the central tube 12 and circumferentially surrounds the central tube 12, thereby forming a liquid storage cavity 16 circumferentially surrounding the air outlet channel 121 to store the aerosol-generating substrate.

In the present application, an axial direction is defined as an extension direction of the central tube 12 (that is, a Z direction in FIG. 1), a width direction of the liquid storage tank housing 10 is defined as a first direction (that is, an X direction in FIG. 1), and a thickness direction of the liquid storage tank housing 10 is defined as a second direction (that is, a Y direction in FIG. 1). The axial direction, the first direction, and the second direction are perpendicular pairwise.

The bottom cover 20 has a shell-like structure with an end open, including a bottom cover bottom wall and a bottom cover sidewall extending from an edge of the bottom cover bottom wall in a same direction. The bottom cover sidewall circumferentially surrounds the bottom cover bottom wall to form a bottom cover accommodating cavity 21 with an end open. An open end of the bottom cover 20 away from the bottom cover bottom wall is inserted into an end of the liquid storage tank housing 10 close to the bottom of the central tube 12. A closed end of the bottom cover 20 provided with the bottom cover bottom wall extends out of the liquid storage tank housing 10. As a preferred implementation, the bottom cover 20 includes a big end 20a and a small end 20b. An outer diameter of the big end 20a is greater than that of the small end 20b to be fitted to the liquid storage tank housing 10, and the small end 20b extends out of the liquid storage tank housing 10.

The sealing member 30 has a ring-like structure, and is arranged around an open end of the bottom cover sidewall of the bottom cover 20, and located between the bottom cover 20 and the liquid storage tank housing 10. The sealing member 30 is configured to seal the liquid storage cavity 16 to prevent leakage of the aerosol-generating substrate in the liquid storage cavity 16 into the bottom cover 20.

The heating unit 40 is housed in a bottom cover accommodating cavity 21 of the bottom cover 20, including a heating shell 41, a sheet-shaped heating element 43, and a liquid guiding member 45. The heating shell 41 is provided with a shell accommodating cavity, the sheet-shaped heating element 43 is housed in the shell accommodating cavity and divides the shell accommodating cavity into a liquid guiding cavity and an airflow channel 47, the end of the liquid guiding member 45 is housed in the liquid guiding cavity and contacts the sheet-shaped heating element 43, another end of the liquid guiding member 45 extends out of the liquid guiding cavity to extend into the liquid storage cavity 16, and the airflow channel 47 communicates with the liquid storage cavity 16 in the liquid storage tank housing 10.

In this way, the liquid guiding member 45 can guide the aerosol-generating substrate in the liquid storage cavity16 to the sheet-shaped heating element 43, the sheet-shaped heating element 43 can heat the aerosol-generating substrate to generate an aerosol, and the aerosol can flow out through the airflow channel 47. Since an aerosol-generating substrate is heated and vaporized by a thinner sheet-shaped heating element 43 in the heating unit 40, the sheet-shaped heating element has a faster heating rate than a tubular heating element, thereby achieving an effect of rapid vaporization. Moreover, top feeding is realized through the liquid guiding member 45, without additionally arranging a liquid inlet channel on a side face of the sheet-shaped heating element 43 to cause the aerosol-generating substrate to enter the liquid guiding member 45, so a distance between the sheet-shaped heating element 43 and an external coil can be reduced, so as to further increase a vaporization speed.

The heating shell 41 includes a heating element base 412 and a heating element fixed cover 414. The heating element base 412 and the heating element fixed cover 414 are snap-fit with each other in the second direction to jointly form the shell accommodating cavity.

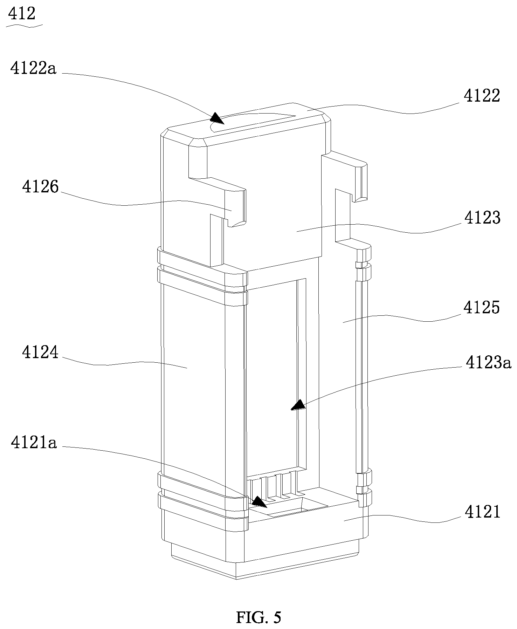

Referring to FIG. 5, specifically, the heating element base 412 includes a base bottom wall 4121, a base top wall 4122, a base rear sidewall, a base front sidewall 4123, a base left sidewall 4124, and a base right sidewall 4125. The base bottom wall 4121 and the base top wall 4122 are spaced apart in the axial direction, the base bottom wall 4121 is located on an end of the heating element base 412 away from the liquid storage cavity 16, and the width of the base top wall 4122 in the second direction is less than that of the base bottom wall 4121 in the second direction, thereby providing an extension space for the liquid guiding member 45. The base rear sidewall and the base front sidewall 4123 are spaced apart in the second direction, the base rear sidewall is connected to the base bottom wall 4121 and a side edge of the base top wall 4122 in the second direction, and the base front sidewall 4123 is connected to the middle of the base bottom wall 4121 in the second direction and another side edge of the base top wall 4122 in the second direction. The base left sidewall 4124 and the base right sidewall 4125 are spaced apart in the first direction and connected between the base bottom wall 4121 and the base top wall 4122, and extend from the base rear sidewall along the second direction to the other side of the base bottom wall 4121 and protrude from the base front sidewall 4123. An end of the base bottom wall 4121 away from the base rear sidewall is provided with a fixed groove 4121a for fixing the heating element fixed cover 414, and a side of the base left sidewall 4124 and a side of the base right sidewall 4125 away from the base rear sidewall are provided with fixed buckles 4126 for fixing the heating element fixed cover 414.

The heating element fixed cover 414 is provided with limit protrusions 4141 at an end in the axial direction and on two sides in the first direction. When the heating element fixed cover 414 is fitted to the heating element base 412, the limit protrusion 4141 at the end of the heating element fixed cover 414 in the axial direction is inserted into the fixed groove 4121a of the base bottom wall 4121, and the limit protrusion 4141 on the two sides in the first direction are engaged with the fixed buckles 4126 on the base left sidewall 4124 and the base right sidewall 4125.

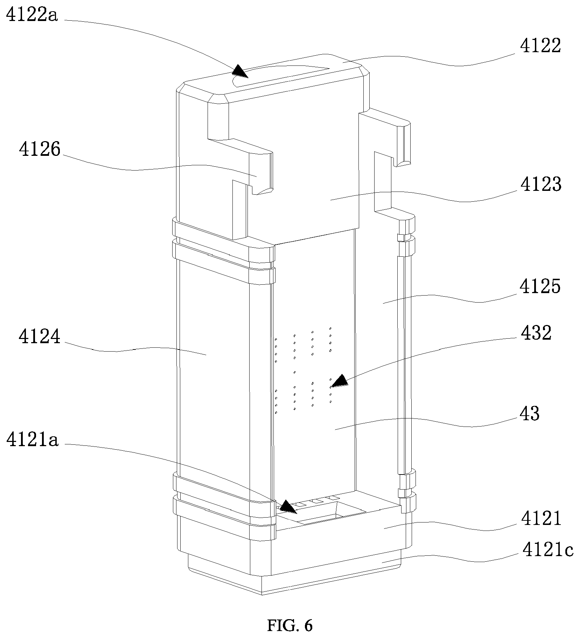

Referring to FIG. 6, the sheet-shaped heating element 43 has a rectangular sheet structure with a thickness ranging from 0.03 mm to 0.15 mm. The base front sidewall 4123 is provided with a communicating groove 4123a throughout the base front sidewall 4123, the sheet-shaped heating element 43 is embedded on a side of the base front sidewall 4123 facing the heating element fixed cover 414 to correspond to the communicating groove 4123a, and the thickness direction of the sheet-shaped heating element 43 extends along the second direction. Therefore, the airflow channel 47 is formed between the base rear sidewall and the sheet-shaped heating element 43, a liquid guiding cavity is formed between the sheet-shaped heating element 43 and the heating element fixed cover 414, and the airflow channel 47 and the liquid guiding cavity are located on two opposite sides of the sheet-shaped heating element 43 in the thickness direction respectively, effectively compressing a size of the heating unit 40 in the second direction.

Further, the sheet-shaped heating element 43 is provided with a plurality of heating element air outlets 432. Each of the heating element air outlets 432 communicates with the liquid guiding cavity and the airflow channel 47. In this way, the aerosol generated by the sheet-shaped heating element 43 by heating the aerosol-generating substrate can flow into the airflow channel 47 through the heating element air outlet 432, thereby following the airflow in the airflow channel 47 to flow out of the heating unit 40.

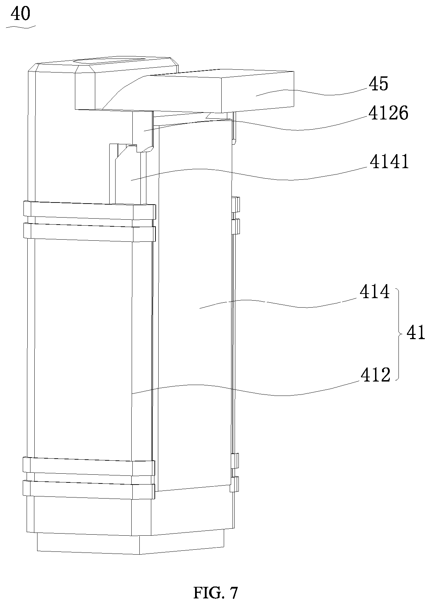

Referring to FIG. 7, the liquid guiding member 45 has a strip-like structure. An end of the liquid guiding member 45 is located in the liquid guiding cavity and clamped between the sheet-shaped heating element 43 and the heating element fixed cover 414, and another end of the liquid guiding member 45 extends out of the liquid guiding cavity from a side of the base top wall 4122 and bends and extends toward a side away from the airflow channel 47 to extend into the liquid storage cavity 16. The liquid guiding member 45 is formed of a porous material such as liquid guide cotton, so as to absorb the aerosol-generating substrate in the liquid storage cavity16 and guide the aerosol-generating substrate to the surface of the sheet-shaped heating element 43.

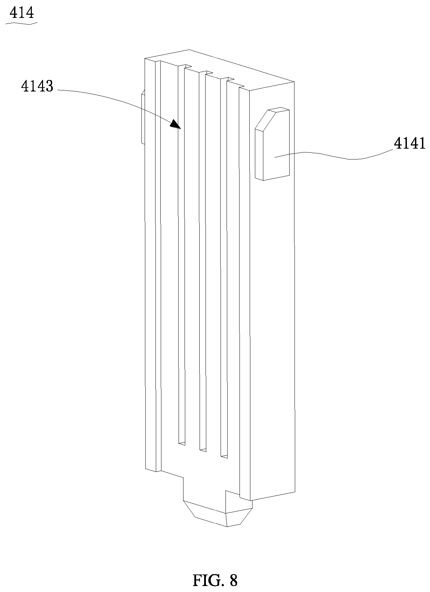

Referring to FIG. 8, preferably, the side of the heating element fixed cover 414 facing the liquid guiding cavity is provided with a plurality of diversion grooves 4143. The plurality of diversion grooves 4143 are spaced apart along the first direction. Each of the diversion grooves 4143 extends along the axial direction, so as to form a capillary phenomenon to improve a liquid guiding effect.

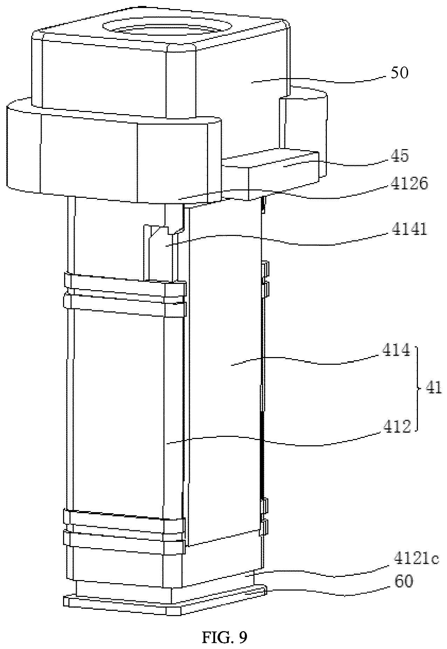

Referring to FIG. 9, in some embodiments, the vaporization assembly 100 further includes an airway sealing member 50. The airway sealing member 50 has a hollow block structure. The airway sealing member 50 is fitted to an end face where the big end 20a and the small end 20b are connected in the bottom cover 20. The central tube 12 in the liquid storage tank housing 10 and the heating element base 412 of the heating unit 40 are inserted to two opposite ends of the airway sealing member 50 respectively. The airway sealing member 50 is provided with a sealing member communication hole 52 communicating with the air outlet channel 121 of the central tube 12 and the airflow channel 47 of the heating unit 40. In this way, the air outlet channel 121 and the airflow channel 47 are sealed by the airway sealing member 50 to ensure sealing performance of an entire airflow path to prevent outflow of the airflow, and at the same time, prevent leakage of the aerosol-generating substrate in the liquid storage cavity 16.

Further, a side of the airway sealing member 50 in the second direction is provided with a sealing member communicating groove 54 communicating with the liquid guiding cavity and the liquid storage cavity 16. Therefore, an end of the liquid guiding member 45 may extend into the liquid storage cavity 16 through the sealing member communicating groove 54.

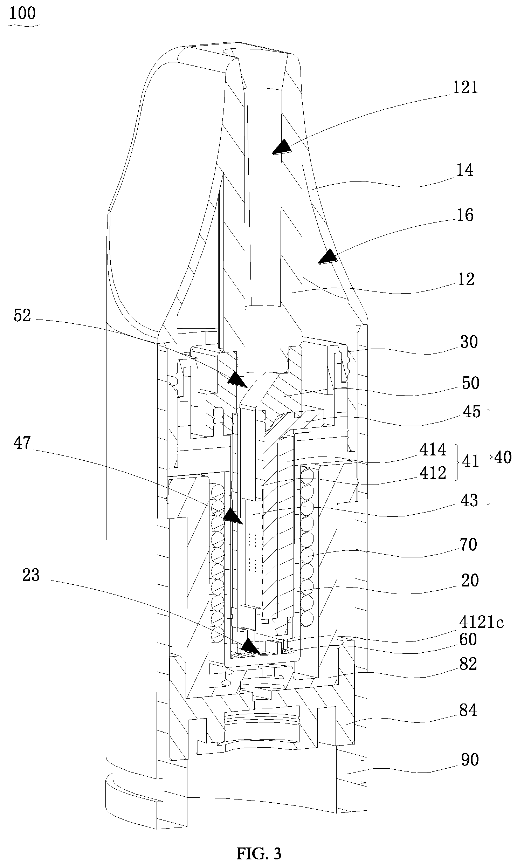

Referring to FIG. 2 and FIG. 3 again, in some embodiments, in order to realize communication between the airflow channel 47 and an external environment, the bottom cover bottom wall of the bottom cover 20 is provided with a bottom cover air inlet 23, the base bottom wall 4121 of the heating element base 412 is provided with a supporting edge 4121c circumferentially extending and protruding therefrom. The supporting edge 4121c may abut against the bottom cover bottom wall and surround the bottom cover air inlet 23. The base bottom wall 4121 is further provided with a shell air inlet 4121b, the shell air inlet 4121b is located on an inner side of the supporting edge 4121c and communicating with the airflow channel 47, and the base top wall 4122 of the heating element base 412 is provided with a shell air outlet 4122a of the airflow channel 47. In this way, airflow outside the bottom cover 20 can flow into the shell air inlet 4121b through the bottom cover air inlet 23 to enter the airflow channel 47 and then flow out through the shell air outlet 4122a.

Further, the vaporization assembly 100 further includes a bottom sealing gasket 60. The bottom sealing gasket 60 has a sheet-shaped structure with a groove in the middle, which is held between the supporting edge 4121c convexly arranged on the base bottom wall 4121 and the bottom cover bottom wall of the bottom cover 20. The bottom sealing gasket 60 plays a sealing role while allowing the base air inlet and the shell air inlet 4121b to communicate with each other, which can prevent infiltration of the aerosol-generating substrate in the liquid storage cavity 16 into the shell air inlet 4121b and the bottom cover air inlet 23.

Still referring to FIG. 2 and FIG. 3, in some embodiments, the vaporization assembly 100 further includes an inductance coil 70, a coil mounting shell 82, a coil mount 84, and a main shell 90.

The inductance coil 70 circumferentially surrounds the bottom cover 20 and extends out of the small end 20b of the liquid storage tank housing 10. The inductance coil 70 generates an alternating magnetic field under the supply of an alternating current, thereby inducing the sheet-shaped heating element 43 to react and generate heat to heat the vaporized aerosol-generating substrate.

The coil mounting shell 82 has a cylindrical structure with an end open. The coil mounting shell 82 sleeves the inductance coil 70 and is fitted to the heating element fixed cover 414, thereby fixing and protecting the inductance coil 70. The coil mount 84 is fitted to an end of the coil mounting shell 82 away from the heating element fixed cover 414 to connect the power supply assembly. The main shell 90 has a cylindrical structure with an end open. The main shell 90 sleeves an end of the liquid storage tank housing 10 to house the coil mounting shell 82 and the coil mount 84.

In the heating unit 40, the vaporization assembly 100, and the electronic vaporizer, the aerosol-generating substrate is heated and vaporized through the sheet-shaped heating element 43 by using the principle of electromagnetic induction, which has higher vaporization efficiency than the tubular heating element. Moreover, the airflow channel 47 and the liquid guiding member 45 are located respectively on the two sides of the heating element in the thickness direction, and top feeding is realized through the liquid guiding member 45, without arranging a liquid inlet channel between the liquid guiding member 45 and the inductance coil 70, which significantly compresses a distance between the heating element and the inductance coil 70 and further improves the vaporization efficiency.

The technical features in the above embodiments may be randomly combined. For concise description, not all possible combinations of the technical features in the above embodiments are described. However, all the combinations of the technical features are to be considered as falling within the scope described in this specification provided that they do not conflict with each other.

## Claims

1. A vaporization assembly (100), comprising a heating unit (40),
wherein the heating unit (40) comprises:
a heating shell (41) provided with a shell accommodating cavity;
a sheet-shaped heating element (43) housed in the shell accommodating cavity and dividing the shell accommodating cavity into a liquid guiding cavity and an airflow channel (47), wherein the liquid guiding cavity and the airflow channel (47) are located respectively on the two sides of the sheet-shaped heating element (43) in the thickness direction; and
a liquid guiding member (45) having one end housed in the liquid guiding cavity and contacting the sheet-shaped heating element (43), and another end extending out of the liquid guiding cavity, and
the vaporization assembly (100) comprises a liquid storage tank housing (10) provided with an air outlet channel (121) and a liquid storage cavity (16) surrounding the air outlet channel (121),
wherein the heating unit (40) is fitted to an end of the liquid storage tank housing (10), the air outlet channel (121) communicates with the airflow channel (47), the liquid guiding cavity communicates with the liquid storage cavity (16), and the end of the liquid guiding member (45) extending out of the liquid guiding cavity bends and enters into the liquid storage cavity (16);
wherein the vaporization assembly (100) comprises a bottom cover (20) for liquid storage tank fitted to an end of the liquid storage tank housing (10),
**characterized in that**
the heating unit (40) is housed in the bottom cover (20);
the vaporization assembly (100) comprises an inductance coil (70) circumferentially surrounding the bottom cover (20), and the inductance coil (70) is adapted to generate an alternating magnetic field under the supply of an alternating current, thereby inducing the sheet-shaped heating element (43) to react and generate heat.

2. The vaporization assembly (100) according to claim 1, wherein the heating shell (41) comprises a heating element base (412) and a heating element fixed cover (414), the heating element fixed cover (414) is located on the side of the liquid guiding member (45) away from the sheet-shaped heating element (43) and fitted to the heating element base (412), the airflow channel (47) is formed between the heating element base (412) and the sheet-shaped heating element (43), and the liquid guiding cavity is formed between the heating element fixed cover (414) and the sheet-shaped heating element (43).

3. The vaporization assembly (100) according to claim 2, wherein the side of the heating element fixed cover (414) facing the liquid guiding cavity is provided with a diversion groove (4143).

4. The vaporization assembly (100) according to claim 1, wherein the sheet-shaped heating element (43) is provided with a heating element air outlet (432) communicating with the liquid guiding cavity and the airflow channel (47).

5. The vaporization assembly (100) according to claim 1, further comprising an airway sealing member (50) fitted between the liquid storage tank housing (10) and the heating unit (40), the airway sealing member (50) communicating with the air outlet channel (121) and the airflow channel (47) of the heating unit (40).

6. The vaporization assembly (100) according to claim 1, further comprising a sealing member (30) for liquid storage tank located between the bottom cover (20) and the liquid storage tank housing (10) to seal the liquid storage cavity (16).

7. The vaporization assembly (100) according to claim 1, wherein the end of the heating shell (41) away from the liquid storage cavity (16) is provided with a shell air inlet (4121b), and the end of the bottom cover (20) away from the liquid storage cavity (16) is provided with a bottom cover air inlet (23) communicating with the shell air inlet (4121b).

8. An electronic vaporizer, comprising the vaporization assembly (100) according to any one of claims 1 to 7, the electronic vaporizer further comprising a power supply assembly electrically connected to the vaporization assembly (100) and configured to supply power to the vaporization assembly (100).

## Patentansprüche

1. Verdampfungsanordnung (100), die eine Heizeinheit (40) umfasst,
wobei die Heizeinheit (40) umfasst:
eine Heizschale (41), die mit einem Schalenaufnahmehohlraum versehen ist;
ein plattenförmiges Heizelement (43), das in dem Schalenaufnahmehohlraum aufgenommen ist und den Schalenaufnahmehohlraum in einen Flüssigkeitsführungshohlraum und einen Luftstromkanal (47) unterteilt, wobei der Flüssigkeitsführungshohlraum und der Luftstromkanal (47) jeweils auf den beiden Seiten des plattenförmigen Heizelements (43) in der Dickenrichtung angeordnet sind; und
ein Flüssigkeitsführungselement (45), das ein Ende, das in dem Flüssigkeitsführungshohlraum aufgenommen ist und das plattenförmige Heizelement (43) berührt, und ein anderes Ende, das sich aus dem Flüssigkeitsführungshohlraum heraus erstreckt, aufweist, und
die Verdampfungsanordnung (100) ein Flüssigkeitsspeichertankgehäuse (10) umfasst, das mit einem Luftauslasskanal (121) und einem Flüssigkeitsspeicherhohlraum (16), der den Luftauslasskanal (121) umgibt, versehen ist,
wobei die Heizeinheit (40) an einem Ende des Flüssigkeitsspeichertankgehäuses (10) eingepasst ist, der Luftauslasskanal (121) mit dem Luftstromkanal (47) in Verbindung steht, der Flüssigkeitsführungshohlraum mit dem Flüssigkeitsspeicherhohlraum (16) in Verbindung steht und sich das Ende des Flüssigkeitsführungselements (45), das sich aus dem Flüssigkeitsführungshohlraum heraus erstreckt, biegt und in den Flüssigkeitsspeicherhohlraum (16) eintritt;
wobei die Verdampfungsanordnung (100) eine Bodenabdeckung (20) für einen Flüssigkeitsspeichertank umfasst, die an einem Ende des Flüssigkeitsspeichertankgehäuses (10) eingepasst ist,
**dadurch gekennzeichnet, dass**
die Heizeinheit (40) in der Bodenabdeckung (20) aufgenommen ist;
die Verdampfungsanordnung (100) eine Induktionsspule (70) umfasst, die die Bodenabdeckung (20) in Umfangsrichtung umgibt, und die Induktionsspule (70) dazu ausgebildet ist, unter der Zufuhr eines Wechselstroms ein magnetisches Wechselfeld zu erzeugen, wodurch das plattenförmige Heizelement (43) dazu gebracht wird, zu reagieren und Wärme zu erzeugen.

2. Verdampfungsanordnung (100) nach Anspruch 1, wobei die Heizschale (41) eine Heizelementbasis (412) und eine feststehende Heizelementabdeckung (414) umfasst, die feststehende Heizelementabdeckung (414) auf der Seite des Flüssigkeitsführungselements (45) angeordnet ist, die von dem plattenförmigen Heizelement (43) entfernt ist, und an der Heizelementbasis (412) eingepasst ist, der Luftstromkanal (47) zwischen der Heizelementbasis (412) und dem plattenförmigen Heizelement (43) ausgebildet ist und der Flüssigkeitsführungshohlraum zwischen der feststehenden Heizelementabdeckung (414) und dem plattenförmigen Heizelement (43) ausgebildet ist.

3. Verdampfungsanordnung (100) nach Anspruch 2, wobei die Seite der feststehenden Heizelementabdeckung (414), die dem Flüssigkeitsführungshohlraum zugewandt ist, mit einer Umlenknut (4143) versehen ist.

4. Verdampfungsanordnung (100) nach Anspruch 1, wobei das plattenförmige Heizelement (43) mit einem Heizelementluftauslass (432) versehen ist, der mit dem Flüssigkeitsführungshohlraum und dem Luftstromkanal (47) in Verbindung steht.

5. Verdampfungsanordnung (100) nach Anspruch 1, die ferner ein Luftwegdichtungselement (50) umfasst, das zwischen dem Flüssigkeitsspeichertankgehäuse (10) und der Heizeinheit (40) eingepasst ist, wobei das Luftwegdichtungselement (50) mit dem Luftauslasskanal (121) und dem Luftstromkanal (47) der Heizeinheit (40) in Verbindung steht.

6. Verdampfungsanordnung (100) nach Anspruch 1, die ferner ein Dichtungselement (30) für einen Flüssigkeitsspeichertank umfasst, das zwischen der Bodenabdeckung (20) und dem Flüssigkeitsspeichertankgehäuse (10) angeordnet ist, um den Flüssigkeitsspeicherhohlraum (16) abzudichten.

7. Verdampfungsanordnung (100) nach Anspruch 1, wobei das Ende der Heizschale (41), das von dem Flüssigkeitsspeicherhohlraum (16) entfernt ist, mit einem Schalenlufteinlass (4121b) versehen ist und das Ende der Bodenabdeckung (20), das von dem Flüssigkeitsspeicherhohlraum (16) entfernt ist, mit einem Bodenabdeckungslufteinlass (23) versehen ist, der mit dem Schalenlufteinlass (4121b) in Verbindung steht.

8. Elektronischer Verdampfer, der die Verdampfungsanordnung (100) nach einem der Ansprüche 1 bis 7 umfasst, wobei der elektronische Verdampfer ferner eine Stromversorgungsanordnung umfasst, die elektrisch mit der Verdampfungsanordnung (100) verbunden ist und dazu ausgebildet ist, die Verdampfungsanordnung (100) mit Strom zu versorgen.

## Revendications

1. Ensemble de vaporisation (100), comprenant une unité de chauffage (40),
dans lequel l'unité de chauffage (40) comprend :
une coque de chauffage (41) munie d'une cavité de logement de coque ;
un élément chauffant en forme de plaquette (43) logé dans la cavité de logement de coque et divisant la cavité de logement de coque en une cavité de guidage de liquide et un canal d'écoulement d'air (47), dans lequel la cavité de guidage de liquide et le canal d'écoulement d'air (47) sont situés respectivement sur les deux côtés de l'élément chauffant en forme de plaquette (43) dans la direction d'épaisseur ; et
un organe de guidage de liquide (45) ayant une extrémité logée dans la cavité de guidage de liquide et en contact avec l'élément chauffant en forme de plaquette (43), et une autre extrémité s'étendant hors de la cavité de guidage de liquide, et
l'ensemble de vaporisation (100) comprend un boîtier de réservoir de stockage de liquide (10) muni d'un canal de sortie d'air (121) et d'une cavité de stockage de liquide (16) entourant le canal de sortie d'air (121),
dans lequel l'unité de chauffage (40) est montée à une extrémité du boîtier de réservoir de stockage de liquide (10), le canal de sortie d'air (121) communique avec le canal d'écoulement d'air (47), la cavité de guidage de liquide communique avec la cavité de stockage de liquide (16), et l'extrémité de l'organe de guidage de liquide (45) s'étendant hors de la cavité de guidage de liquide se plie et pénètre dans la cavité de stockage de liquide (16) ;
dans lequel l'ensemble de vaporisation (100) comprend un couvercle de fond (20) pour réservoir de stockage de liquide monté à une extrémité du boîtier de réservoir de stockage de liquide (10),
**caractérisé en ce que**
l'unité de chauffage (40) est logée dans le couvercle de fond (20) ;
l'ensemble de vaporisation (100) comprend une bobine d'inductance (70) entourant de manière circonférentielle le couvercle de fond (20), et la bobine d'inductance (70) est adaptée pour générer un champ magnétique alternatif sous l'alimentation d'un courant alternatif, induisant ainsi l'élément chauffant en forme de plaquette (43) à réagir et à générer de la chaleur.

2. Ensemble de vaporisation (100) selon la revendication 1, dans lequel la coque de chauffage (41) comprend une base d'élément chauffant (412) et un couvercle fixe d'élément chauffant (414), le couvercle fixe d'élément chauffant (414) est situé du côté de l'organe de guidage de liquide (45) éloigné de l'élément chauffant en forme de plaquette (43) et est monté sur la base d'élément chauffant (412), le canal d'écoulement d'air (47) est formé entre la base d'élément chauffant (412) et l'élément chauffant en forme de plaquette (43), et la cavité de guidage de liquide est formée entre le couvercle fixe d'élément chauffant (414) et l'élément chauffant en forme de plaquette (43).

3. Ensemble de vaporisation (100) selon la revendication 2, dans lequel le côté du couvercle fixe d'élément chauffant (414) faisant face à la cavité de guidage de liquide est muni d'une rainure de déviation (4143).

4. Ensemble de vaporisation (100) selon la revendication 1, dans lequel l'élément chauffant en forme de plaquette (43) est muni d'une sortie d'air d'élément chauffant (432) communiquant avec la cavité de guidage de liquide et le canal d'écoulement d'air (47).

5. Ensemble de vaporisation (100) selon la revendication 1, comprenant en outre un organe d'étanchéité de voie d'air (50) monté entre le boîtier de réservoir de stockage de liquide (10) et l'unité de chauffage (40), l'organe d'étanchéité de voie d'air (50) communiquant avec le canal de sortie d'air (121) et le canal d'écoulement d'air (47) de l'unité de chauffage (40).

6. Ensemble de vaporisation (100) selon la revendication 1, comprenant en outre un organe d'étanchéité (30) pour réservoir de stockage de liquide situé entre le couvercle de fond (20) et le boîtier de réservoir de stockage de liquide (10) pour étancher la cavité de stockage de liquide (16).

7. Ensemble de vaporisation (100) selon la revendication 1, dans lequel l'extrémité de la coque de chauffage (41) éloignée de la cavité de stockage de liquide (16) est munie d'une entrée d'air de coque (4121b), et l'extrémité du couvercle de fond (20) éloignée de la cavité de stockage de liquide (16) est munie d'une entrée d'air de couvercle de fond (23) communiquant avec l'entrée d'air de coque (4121b).

8. Vaporisateur électronique, comprenant l'ensemble de vaporisation (100) selon l'une quelconque des revendications 1 à 7, le vaporisateur électronique comprenant en outre un ensemble d'alimentation électrique connecté électriquement à l'ensemble de vaporisation (100) et configuré pour alimenter en courant l'ensemble de vaporisation (100).
